# FASCICULE DE BREVET EUROPEEN

(11) **EP 2 984 075 B1**
(45) Date de publication et mention de la délivrance du brevet: **26.09.2018**
(21) Numéro de dépôt: 14720659.3
(22) Date de dépôt: 04.04.2014
(51) Int. Cl.: C07D 249/12, C07D 249/14

(54) **OBTENTION DE SOLUTIONS D'OTA EN MILIEU ACIDE SULFURIQUE CONCENTRÉ; LESDITES SOLUTIONS; PRÉPARATION D'ONTA**
VERFAHREN ZUR GEWINNUNG VON OTA-LÖSUNGEN IN EINEM KONZENTRIERTEN SCHWEFELSÄUREMEDIUM, DERARTIGE LÖSUNGEN UND VERFAHREN ZUR HERSTELLUNG VON ONTA
METHOD FOR OBTAINING SOLUTIONS OF OTA IN A CONCENTRATED SULPHURIC ACID MEDIUM; SAID SOLUTIONS; AND METHOD FOR PREPARING ONTA

(30) Priorité: 08.04.2013 FR 1300801
(43) Date de publication de la demande: 17.02.2016
(73) Titulaire: ARIANEGROUP SAS, 75015 Paris (FR); Eurenco, 91300 Massy (FR)
(72) Inventeur: HERVE, Grégoire, 75012 Paris (FR); JACOB, Guy, F-91710 Vert Le Petit (FR); CAGNON, Guy, F-91610 Ballancourt Sur Essonne (FR); BOUCHEZ, Jean-marc, F-91610 Ballancourt Sur Essonne (FR)
(74) Mandataire: Le Roux, Martine
(86) Numéro de dépôt international: PCT/FR2014/050817
(87) Numéro de publication internationale: WO 2014/167226

(56) Documents cités:
- US-B1- 6 583 293
- KONSTANTINOVA I D ET AL: "Chemoenzymatic method of 1,2,4-triazole nucleoside synthesis: Possibilities and limitations", RUSSIAN JOURNAL OF BIOORGANIC CHEMISTRY, SP MAIK NAUKA/INTERPERIODICA, DORDRECHT, vol. 39, no. 1, 23 janvier 2013 (2013-01-23), pages 53-71, XP035170112, ISSN: 1608-330X, DOI: 10.1134/S1068162013010056
- CHIPEN, G I: METODY POLUCHENIYA KHIMICHESKIKH REAKTIVOV I PREPARATOV, vol. 14, 1964, pages 119-120, XP008166548,

## Description

La présente invention a été conçue et développée dans le contexte de la préparation du 3-nitro-1,2,4-triazol-5-one (couramment appelé oxynitrotriazole ou ONTA). Ce composé présente l'énorme avantage de posséder des performances explosives (compte tenu de sa masse volumique) voisines de celles de l'hexogène, tout en exhibant une sensibilité aussi faible que celle de la tolite (bien inférieure à celle dudit hexogène, et à celle de l'octogène).

La présente invention a plus particulièrement pour objet :
- un procédé d'obtention original de solutions renfermant du 1,2,4-triazol-5-one (couramment appelé oxytriazole ou OTA) dans de l'acide sulfurique concentré ;
- lesdites solutions (portant des signatures de leur procédé d'obtention original), et
- un procédé de préparation dudit 3-nitro-1,2,4-triazol-5-one (ONTA) à partir de telles solutions.

L'obtention de ces solutions à partir du 3-amino-1,2,4-triazole (couramment appelé aminotriazole ou ATA), et leur utilisation pour la préparation dudit 3-nitro-1,2,4-triazol-5-one (ONTA) (en fait l'utilisation dudit 3-amino-1,2,4-triazole (ATA) comme précurseur du 1,2,4-triazol-5-one (OTA) pour préparer ledit 3-nitro-1,2,4-triazol-5-one (ONTA)) constituent la clé de la présente invention.

### ART ANTERIEUR

Ledit 3-nitro-1,2,4-triazol-5-one (ONTA) est classiquement obtenu en 2 étapes :
- une première étape de préparation du 1,2,4-triazol-5-one (OTA) ; et
- une seconde étape de nitration dudit 1,2,4-triazol-5-one (OTA) : OTA → ONTA.

Il existe plusieurs voies d'accès audit 1,2,4-triazol-5-one (OTA). Il existe plusieurs modes de mise en oeuvre de sa nitration.

Pour la préparation du 1,2,4-triazol-5-one (OTA), on a notamment décrit :
- la réaction du carbamate d'éthyl éthoxyméthylène avec l'hydrazine,
- la réaction du carbohydrazide avec l'orthoformate d'éthyle,
- la décarboxylation de l'acide 3-hydroxy-1,2,4-triazol-5-carboxylique,
- la réaction du 3-chloro-1,2,4-triazole avec la soude caustique, et
- la réaction du chlorhydrate de semicarbazide (ou de la base libre semicarbazide) avec l'acide formique.

Cette dernière voie d'accès à l'OTA est largement préférée. Elle est développée au stade industriel.

Sa mise en oeuvre, suivie de celle d'une nitration avec de l'acide nitrique, est notamment décrite dans la demande de brevet EP 0 210 881 ainsi que dans le brevet US 4 733 610. Selon l'enseignement de ladite demande EP 0 210 881, la réaction du chlorhydrate de semicarbazide avec l'acide formique est mise en oeuvre en milieu aqueux, pendant 6 à 8 heures, à une température de 85-90°C. L'OTA, obtenu avec un rendement de l'ordre de 80 %, est isolé. Sa nitration est ensuite mise en oeuvre dans de l'acide nitrique à 98 % en masse. Selon l'enseignement du brevet US 4 733 610, l'OTA obtenu n'est pas isolé. Il est nitré avec de l'acide nitrique à 70 % en masse.

Le brevet US 4 927 940 décrit également la préparation de l'OTA par réaction du chlorhydrate de semicarbazide avec l'acide formique, ledit chlorhydrate de semicarbazide ayant été préparé *in situ* par réaction d'hydrazine et d'urée.

La demande de brevet GB 2 218 986, le document « United States Statutory Invention Registration » H 861 et le brevet US 6 583 293 décrivent eux différents modes de mise en oeuvre de la nitration de l'OTA, notamment, respectivement, avec de l'acide nitrique concentré (90 % en masse), dans de l'acide nitrique moins concentré (70 % en masse) et en milieu sulfonitrique (H₂SO₄ 98 % + HNO₃ 70% en masse).

### INVENTION

Dans un tel contexte, les inventeurs ont cherché et optimisé un procédé de préparation du 3-nitro-1,2,4-triazol-5-one (ONTA) alternatif aux procédés existants, particulièrement performant. Ce procédé, qui comprend les deux étapes du type indiqué ci-dessus : préparation de l'OTA et nitration dudit OTA, est décrit ci-après. Il est particulièrement performant, intéressant, en ce que :
- les produits utiles à sa mise en oeuvre (voir plus loin) sont tous des produits industriels et peu onéreux. En effet, l'homme du métier n'ignore pas que le semicarbazide, sous forme libre ou sous forme de chlorhydrate, à partir duquel l'OTA est donc généralement obtenu (voir ci-dessus), est un produit onéreux ;
- ses deux étapes principales (obtention de l'OTA (plus précisément de solutions d'OTA (voir ci-après) et nitration de celui-ci) sont quantitatives (les taux de transformation ATA → OTA → ONTA peuvent sans difficultés particulières être de 100 %). Il n'y a par ailleurs pas de difficultés particulières à les mettre en oeuvre, y compris au stade industriel ; et
- ses deux étapes principales (voir ci-dessus) peuvent être mises en oeuvre, successivement, au sein d'un unique réacteur. Ce mode opératoire (« one pot ») est particulièrement avantageux.

Selon son premier objet, la présente invention concerne donc un procédé d'obtention d'une solution de 1,2,4-triazol-5-one (OTA) dans de l'acide sulfurique concentré. Un tel procédé, original, constitue, comme indiqué ci-dessus et précisé ci-après, la clé de la présente invention.

De façon caractéristique, ledit procédé comprend :
a) la préparation, dans de l'acide sulfurique concentré, de l'hydrogénosulfate de diazonium de formule : 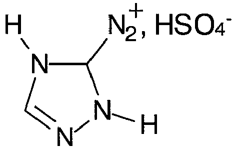 par réaction, à froid, dans de l'acide sulfurique concentré, d'au moins un nitrite sur le 3-amino-1,2,4-triazole ; et
b) le traitement thermique du milieu réactionnel renfermant ledit hydrogénosulfate de diazonium pour l'obtention de ladite solution.

De façon caractéristique, le 3-amino-1,2,4-triazole (ATA ; produit commercial bon marché, notamment utilisé comme herbicide) est utilisé comme matière première (comme précurseur de l'OTA). De façon caractéristique, il est mis à réagir à froid avec (au moins) un nitrite en milieu acide sulfurique concentré, i.e. il subit une diazotation (étape a ci-dessus).

Le milieu acide sulfurique concentré (H₂SO₄ / H₂O) est opportunément aussi concentré que possible. L'acide sulfurique employé renferme ainsi généralement au moins 75 % en masse, avantageusement au moins 80 % en masse, et très avantageusement au moins 95 % en masse, d'acide sulfurique. Opportunément, l'acide sulfurique employé est de l'acide sulfurique à 98 % en masse. Un tel acide sulfurique concentré existe commercialement.

La concentration en ATA dans ledit acide sulfurique concentré est généralement comprise entre 0,3 et 2 mol/l. elle est avantageusement comprise entre 1,3 et 1,6 mol/l. Elle est très avantageusement comprise entre 1,4 et 1,5 mol/l. Il est évidemment vivement opportun que l'ATA présent le soit à l'état dissous. La concentration en ATA est par ailleurs opportunément limitée en référence à la mise en oeuvre du traitement thermique ultérieur. En effet, la décomposition de l'hydrogénosulfate de diazonium intermédiaire impliquant le dégagement d'azote (i.e. la formation de mousse), il convient d'être capable de maîtriser ce dégagement gazeux.

Ledit au moins un nitrite intervenant intervient donc comme agent de diazotation. Il peut notamment être choisi parmi les nitrites organiques (tels les nitrites d'alkyle et notamment le nitrite d'isopentyle), les nitrites d'alcalins et les nitrites d'alcalino-terreux. Il consiste avantageusement en le nitrite de sodium (NaNO₂) et/ou le nitrite de potassium (KNO₂). Il consiste très avantageusement en le nitrite de sodium (NaNO₂). La réaction de diazotation de l'ATA est en fait généralement mise en oeuvre en présence d'un unique nitrite ; ledit unique nitrite consiste donc très avantageusement en le nitrite de sodium (NaNO₂).

Le rapport molaire nitrite(s) / 3-amino-1,2,4-triazole (ATA), nitrite / 3-amino-1,2,4-triazole (ATA) le plus souvent, est généralement compris entre 1 et 2, avantageusement entre 1,2 et 1,4. Il est en fait opportun de limiter la quantité de nitrite(s) intervenant, pour limiter, d'une part, le dégagement ultérieur (lors du traitement thermique) de gaz (NO), suite à la décomposition du nitrite en excès, et d'autre part, la quantité de sel présente dans le milieu réactionnel, i.e. présente dans la solution d'OTA obtenue, en référence à la pureté de l'ONTA préparé à partir d'une telle solution (voir ci-après).

Ledit au moins un nitrite est mis à réagir sur l'ATA à froid (en référence à la stabilité de l'hydrogénosulfate de diazonium intermédiaire). Il est généralement mis à réagir à une température entre 0 et 10°C, avantageusement entre 2 et 7°C. Pour éviter tout échauffement excessif du milieu réactionnel, du fait de la réaction (de diazotation) exothermique en cause, on préconise vivement de mettre progressivement en contact les réactifs, généralement d'introduire progressivement le(s) nitrite(s) dans la solution d'acide sulfurique renfermant l'ATA. Typiquement, la réaction de diazotation de l'ATA est mise en oeuvre à 5°C.

Pour l'obtention de l'OTA, plus précisément des solutions d'OTA selon l'invention (en milieu acide sulfurique concentré), l'hydrogénosulfate de diazonium (dont la formule est indiquée ci-dessus) obtenu par diazotation de l'ATA (étape a explicitée ci-dessus) est traité thermiquement (étape b).

Ce traitement thermique assure l'obtention de l'OTA, *via* l'élimination du groupement diazo (voir le schéma réactionnel ci-après). L'élimination du groupement diazo implique le dégagement d'azote. Ce dégagement est opportunément maitrisé pour éviter tout débordement (voir ci-dessus). Le traitement thermique implique aussi la décomposition du nitrite en excès, i.e. le dégagement de NO (voir ci-dessus).

Afin de maitriser ledit traitement thermique, il est ainsi vivement préconisé de le mettre en oeuvre selon deux phases successives : une phase de montée en température lente avec contrôle du dégagement gazeux (principalement de l'azote) (voir ci-dessus) puis une phase de maintien à haute température pour l'obtention de l'OTA. Ledit traitement thermique est donc avantageusement mis en oeuvre selon les deux phases ci-après :
- une première phase de montée en température, mise en oeuvre avec contrôle du dégagement gazeux, de façon à éviter tout débordement, jusqu'à une température T d'au moins 50°C, avantageusement en deçà de 120°C, très avantageusement jusqu'à une température T de 100°C, et
- une seconde phase de maintien à ladite température T.

Pour assurer un tel contrôle du dégagement gazeux, la montée en température de ladite première phase n'est pas trop rapide ; elle est avantageusement mise en oeuvre selon une rampe de 9°C/h à 16°C/h, très avantageusement selon une rampe de 12°C/h.

Au sein de solutions d'acide sulfurique concentrées, l'OTA est donc obtenu, selon l'invention, à partir de l'ATA, par a) diazotation, ladite diazotation conduisant à l'hydrogénosulfate de diazonium dont la formule a été donnée ci-dessus, puis b) traitement thermique ; ledit traitement thermique comprenant l'élimination du groupement diazo dudit hydrogénosulfate de diazonium et la conversion du groupement hydrogénosulfate (HSO₄⁻) dudit hydrogénosulfate (voir le schéma réactionnel ci-après).

L'obtention des solutions de 1,2,4-triazol-5-one (OTA) selon l'invention est donc mis en oeuvre selon le schéma réactionnel ci-après. Ledit schéma réactionnel est montré, de façon nullement limitative, avec l'utilisation du nitrite de sodium, comme nitrite de diazotation.

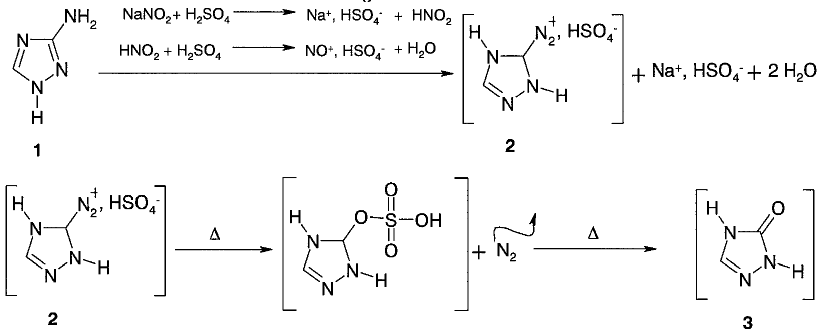

L'obtention desdites solutions de 1,2,4-triazol-5-one (OTA) selon l'invention est très avantageusement mise en oeuvre dans les conditions ci-après :
a) H₂SO₄ concentré : 98 % en masse,
   ATA : 1,43 mol/l,
   rapport molaire NaNO₂/ ATA : 1,27, et
   température de réaction : 5°C ;
b) chauffage jusqu'à 100°C, à raison de 12°C/h, puis
   maintien à 100°C pendant 2 heures.

Selon son deuxième objet, la présente invention concerne les solutions de 1,2,4-triazol-5-one (OTA) dans de l'acide sulfurique concentré, susceptibles d'être obtenues par le procédé décrit ci-dessus, premier objet de la présente invention, ladite solution renfermant au moins un hydrogénosulfate, autre que l'hydrogénosulfate de diazonium.

Lesdites solutions sont *per se* nouvelles en ce qu'elles portent des signatures originales dudit procédé d'obtention.

Elles renferment au moins un sel de nature originale : hydrogénosulfate(s) du(des) cation(s) du(des) nitrite(s) utilisé(s) comme agent de diazotation (voir le au moins un nitrite mentionné ci-dessus), hydrogénosulfate de sodium dans un contexte d'utilisation de nitrite de sodium. Lesdites solutions renferment donc au moins un hydrogénosulfate, autre que l'hydrogénosulfate de diazonium : l'hydrogénosulfate du cation du nitrite mis à réagir avec le 3-amino-1,2,4-triazole ou les hydrogénosulfates des cations des nitrites mis à réagir avec le 3-amino-1,2,4-triazole (dans l'hypothèse où plusieurs nitrites ont été mis à réagir).

Elles renferment par ailleurs généralement de l'ATA, plus généralement seulement des traces d'ATA (au vu de la conversion quantitative dudit ATA en OTA selon le procédé de l'invention).

Elles ne renferment plus de nitrite(s), même ajouté(s) en excès, dans la mesure où le traitement thermique en a assuré la décomposition (voir le dégagement de NO mentionné ci-dessus).

De telles solutions se distinguent assurément de solutions similaires qui seraient obtenues avec de l'OTA préparé à partir du chlorhydrate de semicarbazide, par l'absence de tout produit chloré en leur sein.

Selon son troisième objet, la présente invention concerne un procédé de préparation du 3-nitro-1,2,4-triazol-5-one (ONTA), qui, de façon originale, utilisent les solutions d'OTA dans l'acide sulfurique concentré décrites ci-dessus (solutions qui constituent le second objet de la présente invention, un mode d'obtention de telles solutions constituant le premier objet de la présente invention).

Ledit procédé de préparation comprend, de façon originale, la nitration de telles solutions (solutions d'OTA en milieu acide sulfurique concentré, obtenues à partir d'ATA, d'acide sulfurique concentré et d'au moins un nitrite), plus exactement la nitration de l'OTA présent au sein de telles solutions.

Le procédé de préparation de l'ONTA selon l'invention comprend donc, de façon caractéristique :
α) la mise à disposition d'une solution de 1,2,4-triazol-5-one (OTA) dans de l'acide sulfurique concentré selon le deuxième objet de la présente invention ou l'obtention d'une solution de 1,2,4-triazol-5-one (OTA) par le procédé constituant le premier objet de la présente invention (on a alors l'étape α = étape a + étape b ci-dessus) ;
   puis
β) la nitration dudit 1,2,4-triazol-5-one (OTA) par addition d'acide nitrique concentré à ladite solution.

En référence à l'étape α ci-dessus, on a compris que les deux alternatives sont énoncées dans la mesure où les solutions en cause sont *per se* nouvelles.

Lesdites solutions sont généralement obtenues par la mise en oeuvre desdites étapes a et b, précisées ci-dessus, du procédé, premier objet de la présente invention. Ainsi, dans cette hypothèse, le procédé de préparation de l'ONTA selon l'invention peut-il être schématisé comme suit, avec utilisation du nitrite de sodium, comme agent de nitration. Bien évidemment, la diazotation peut être mise en oeuvre avec un autre nitrite (voir ci-dessus).

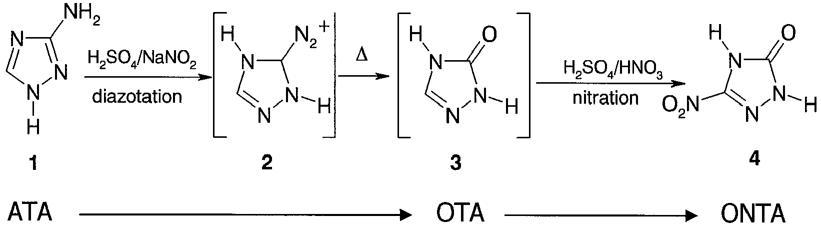

Notons d'ores et déjà ici que la mise en oeuvre de l'étape α = a + b (ATA → OTA) peut être totalement dissociée de celle de l'étape β (nitration : OTA → ONTA), aussi bien en termes de localisation que de temps.

Pour ce qui concerne la nitration (étape β), elle est avantageusement mise en oeuvre dans les conditions ci-après :
- avec un acide nitrique (HNO₃ / H₂O), aussi concentré que possible. Ledit acide nitrique concentré renferme au moins 75 % en masse, avantageusement au moins 80 % en masse, très avantageusement à au moins 95 % en masse, d'acide nitrique. On utilise avantageusement un acide nitrique à 98 % en masse. Un tel acide nitrique concentré existe commercialement ;
- avec un rapport molaire HNO₃ / 1,2,4-triazol-5-one (OTA) compris entre 1,9 et 2,5, avantageusement entre 1,95 et 2,2. Les inventeurs ont montré qu'il n'y avait aucun intérêt à utiliser de grandes quantités d'acide nitrique ;
- à une température comprise entre 50 et 70°C. A une telle température, la nitration est généralement complète en une durée de 1 h 30 à 2 h 30. En deçà de 50°C, la cinétique de la réaction de nitration est trop lente ; au-delà de 70°C, l'acide nitrique se décompose : l'apparition de vapeurs rousses en témoigne. Il est avantageux de mettre en oeuvre la nitration à une température de 65°C.

Pour une bonne conduite de la réaction de nitration (au sein d'un milieu homogène, avec stabilisation de la température), il est vivement préconisé d'ajouter aux solutions « à nitrer» de l'eau (H₂O). On préconise d'ajouter aux dites solutions, avant la mise en oeuvre de la nitration, de 10 à 20 % en masse d'eau, avantageusement 15 % en masse d'eau.

A partir des solutions d'OTA de l'invention (solutions dans l'acide sulfurique concentré), la préparation d'ONTA est très avantageusement mise en oeuvre dans les conditions ci-après :
addition (préalable) de 15 % en masse d'eau,
HNO₃ concentré : 98 % en masse,
rapport molaire HNO₃/ 1,2,4-triazol-5-one (OTA) : 1,97, et
température de nitration : 65°C.

On a indiqué ci-dessus que la mise en oeuvre de la nitration (étape β) peut être totalement dissociée de celle de la mise à disposition ou de la préparation de la solution à nitrer (étape α, avec généralement α = a + b). On peut, de manière générale, prévoir un stockage, plus ou moins long, de la solution obtenue, avant la mise en oeuvre de la nitration.

Par ailleurs, la solution à nitrer peut tout à fait être obtenue sur un site différent de celui de sa nitration, sur un même site mais dans un autre réacteur (décalage dans l'espace, de plus ou moins grande amplitude, qui *a priori* implique un décalage dans le temps conséquent).

Selon une autre variante, de loin préférée, le procédé de préparation de l'ONTA, selon l'invention, comprend :
- une première étape d'obtention d'une solution du 1,2,4-triazol-5-one (OTA) dans de l'acide sulfurique concentré selon le procédé décrit ci-dessus (étape α = étape a + étape b), et
- une seconde étape de nitration dudit 1,2,4-triazol-5-one (OTA) par addition d'acide nitrique concentré à ladite solution (étape β) ; lesdites première et seconde étapes étant mises en oeuvre au sein d'un même réacteur.

L'OTA est ainsi préparé *in situ* pour sa nitration. Les opérations d'obtention de l'OTA et de nitration dudit OTA sont mises en oeuvre dans le même réacteur. Le procédé en cause est un procédé « one pot ». L'homme du métier n'ignore pas l'intérêt de ce type de procédé, notamment pour sa mise en oeuvre à l'échelle industrielle.

Selon cette variante de mise en oeuvre préférée du nouveau procédé de préparation de l'ONTA de l'invention, on met donc successivement (avec décalage ou pas dans le temps, généralement sans décalage dans le temps) en oeuvre, au sein d'un même réacteur, les réactions explicitées ci-dessus : ATA → OTA → ONTA.

Quelle que soit enfin la variante exacte de mise en oeuvre du procédé de préparation de l'ONTA selon l'invention (mode opératoire exact ; étapes α et β mises en oeuvre sur un même site ou pas, dans un même réacteur ou pas, avec décalage dans le temps ou pas), il est généralement opportun de récupérer ledit ONTA, obtenu donc en milieu sulfurique concentré.

Pour une telle récupération, il est préconisé une précipitation (cristallisation), suite à un abaissement de la température du milieu réactionnel, suivie d'une filtration. Ladite filtration est elle-même avantageusement suivie d'un essorage des cristaux récupérés.

Pour l'obtention d'un produit (précipité, cristallisé) aussi pur que possible, on vise une précipitation sélective vis-à-vis des sels présents (principalement de l'(des) hydrogénosulfate(s) présent(s)). Pour la mise en oeuvre d'une telle précipitation sélective, il est recommandé d'ajouter de l'eau au milieu réactionnel (renfermant ledit ONTA) refroidi (en cours de refroidissement et/ou à l'issue du refroidissement, généralement à l'issue dudit refroidissement), ladite eau contribuant au maintien en solution des sels présents (du(des) hydrogénosulfate(s) présent(s)). L'addition de 8 à 15 % en masse, de préférence de 10 % en masse, d'eau est vivement préconisée.

Le refroidissement du milieu réactionnel est par ailleurs avantageusement conduit à (seulement) une température entre 8 et 12°C, avantageusement à 10°C. Les inventeurs ont montré qu'un refroidissement à des températures inférieures conduisait à la précipitation d'un ONTA moins pur.

La sélectivité de la précipitation peut en fait être optimisée du fait de la présence d'eau, en quantité suffisante (voir ci-dessus) et si le refroidissement est mise en oeuvre à (seulement) une température entre 8 et 12°C, avantageusement à 10°C.

L'homme du métier a d'ores et déjà relevé tout l'intérêt de l'invention, tous ses avantages. Comme indiqué dans l'introduction du présent texte, la présente invention permet l'obtention de l'ONTA sans faire appel à des produits couteux (seulement à l'ATA, à au moins un nitrite et à des solutions concentrés d'H₂SO₄ et de HNO₃), *via* deux étapes (obtention de l'OTA, nitration de l'OTA) quantitatives (des taux de transformation de 100 % peuvent sans difficultés particulières être obtenus), qui, opportunément, peuvent être mises en oeuvre dans un unique réacteur.

Par ailleurs, le procédé de l'invention, avec notamment l'addition d'eau en phase de récupération de l'ONTA (pour maintenir en solution le(s) sel(s) formé(s), de par l'intervention d'au moins un nitrite (agent de diazotation de l'ATA) en amont), permet d'obtenir de l'ONTA de très grande pureté (98 % en masse, généralement après essorage (i.e. débarrassé des eaux mères)). En référence à la pureté de l'ONTA finalement récupéré, on peut ajouter ce qui suit. Si l'on précipite le maximum d'ONTA (en vue d'« optimiser » le rendement final de la réaction), on précipite malheureusement conjointement, au moins en partie, les impuretés présentes dans le milieu (sel(s) d'hydrogénosulfate). Si l'on précipite sélectivement l'ONTA (avec donc un degré de pureté « optimal »), c'est au détriment du rendement. Un compromis intéressant peut être trouvé. Le taux de pureté (98 % en masse) indiqué ci-dessus a été obtenu avec un rendement de 55 % (ONTA sec final/ ATA de départ).

### EXEMPLE ET FIGURES

On se propose maintenant d'illustrer, de façon nullement limitative, les différents aspects de l'invention par l'exemple ci-après et les figures annexées.
La figure 1 est un organigramme (« flow-sheet ») d'une variante de mise en oeuvre (« one pot ») du procédé de préparation de l'ONTA selon l'invention, variante sans stockage intermédiaire de la solution d'OTA dans de l'acide sulfurique concentré, obtenue dans un premier temps.
Les figures 2 à 4 sont des courbes représentant respectivement :
   - pour la figure 2, l'augmentation du rendement en OTA lors de la montée en température du réacteur (étape b (traitement thermique) de l'exemple ci-après) ;
   - pour la figure 3, l'augmentation du rendement en OTA, dans les mêmes conditions de montée en température, mais au sein de solutions d'acide sulfurique, plus ou moins concentrées (44 %, 81 % et 98 % en masse),
   - pour la figure 4, l'augmentation du rendement en ONTA en fonction du temps, après la coulée de l'acide nitrique (lors donc de la mise en oeuvre de la nitration : étape β de l'exemple ci-après).

Des précisions sont données au sujet de ces courbes dans l'exemple ci-après.

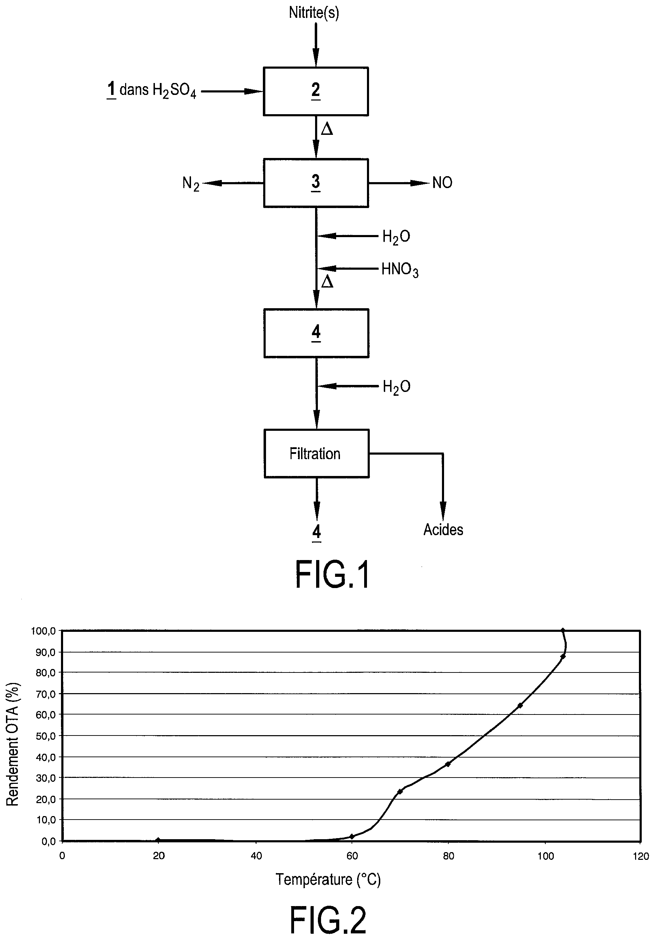

Pour ce qui concerne la figure 1, on a indiqué, comme cela a déjà été fait dans les schémas réactionnels donnés en amont dans la description :
en 1, le 3-amino-1,2,4-triazole = ATA,
en 2, l'hydrogénosulfate de diazonium (= « ATA diazoté »),
en 3, le 1,2,4-triazol-5-one = OTA, et
en 4, le 3-nitro-1,2,4-triazol-5-one = ONTA.

On comprend que le au moins un nitrite est mis à réagir sur 1 dans l'acide sulfurique pour le diazoter.

Le traitement thermique (Δ) assure l'élimination du groupement diazo (d'où le dégagement de N₂), il décompose quelque peu le nitrite qui n'a pas réagi (d'où le dégagement de NO) et permet l'obtention de 3.

Cette première partie de l'organigramme correspond au procédé d'obtention d'une solution de 3 dans l'acide sulfurique concentré (premier objet de la présente invention).

La seconde partie de l'organigramme correspond à la préparation et à la récupération de 4 (troisième objet de la présente invention).

La nitration (avec chauffage : Δ) est mise en oeuvre dans des conditions « optimales » du fait de l'ajout d'H₂O, avant celui de l'acide nitrique.

Pour la récupération (par précipitation) de 4, «pollué a minima » par l'hydrogénosulfate ou les hydrogénosulfates présents, on refroidit à une température entre 8 et 12 °C et on ajoute à nouveau de l'eau.

L'ONTA précipité (cristallisé) est récupéré par filtration. On récupère les vieux acides (H₂SO₄ + HNO₃) qui peuvent être séparés. L'acide nitrique est avantageusement recyclé.

Le procédé exemplifié, tel que schématisé sur la figure 1, est un procédé « one pot ».

### Exemple

### A. Synthèse de l'OTA : obtention d'une solution d'OTA dans de l'acide sulfurique concentré

A 1,2 L d'acide sulfurique concentré (acide sulfurique commercial concentré à 98 % en masse), préalablement refroidi à 5°C, sont additionnés 144 g (1,714 mol) de 3-amino-1,2,4-triazole (ATA commercial).

Le mélange est refroidi à 5°C.

A cette température de 5°C, 150 g (2,174 mol) de nitrite de sodium (NaNO₂) sont progressivement ajoutés. On veille à ne pas dépasser 10°C.

En fin d'introduction du nitrite de sodium, le milieu réactionnel est chauffé progressivement sous une rampe de température de 12°C/h, jusqu'à 100°C. Une fois cette température de 100°C atteinte (au bout d'environ 8 heures), le milieu réactionnel est laissé 2 heures sous agitation (à ladite température de 100°C).

L'avancement de la réaction est contrôlé par chromatographie liquide à haute pression (HPLC), après étalonnage externe (à l'aide d'un OTA commercial). On mesure le rendement en OTA sur des échantillons prélevés à différentes températures (dilués dans de l'eau pour bloquer la réaction).

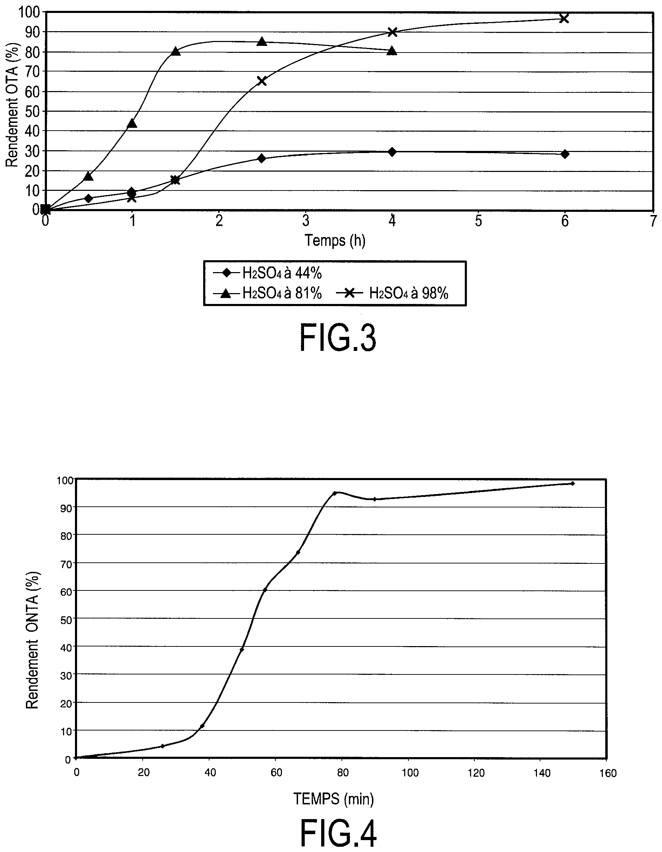

On a ainsi établi la figure 2 (on voit qu'un rendement de 100 % peut être atteint) et la courbe : H₂SO₄ 98 % en masse de la figure 3.

Cette même synthèse et ces mêmes mesures ont été mises en oeuvre dans les mêmes conditions avec des acides sulfuriques moins concentrés (voir les deux autres courbes de la figure 3 : H₂SO₄ 44 % en masse et H₂SO₄ 81 % en masse).

La figure 3 montre à l'évidence l'intérêt de mettre en oeuvre la réaction avec de l'acide sulfurique aussi concentré que possible.

La solution obtenue peut être utilisée directement pour la préparation de l'ONTA.

Elle peut aussi être stockée et/ou transportée pour servir de matière première à une synthèse ultérieure de l'ONTA.

Dans le cadre de la variante de mise en oeuvre exemplifiée, elle a été utilisée, de suite, pour la synthèse (« one pot ») de l'ONTA.

### B. Synthèse et récupération de l'ONTA

A la solution d'OTA obtenue (dans l'acide sulfurique à 98 %), sont ajoutés 509 g (28,28 mol) d'eau sous agitation, puis 213 g (3,38 mol) d'acide nitrique concentré (acide nitrique commercial à 98 % en masse).

Après introduction de l'acide nitrique, le milieu est porté à 65°C et laissé 2 heures sous agitation à cette température.

Des échantillons sont prélevés pour suivre l'avancement de la nitration (une fois que la température du milieu réactionnel a atteint 65°C). Lesdits échantillons sont analysés après étalonnage externe (à l'aide d'un ONTA commercial). Les résultats sont montrés sur la figure 4. On voit qu'un très haut rendement peut être obtenu.

Pour la récupération de l'ONTA préparé, on a procédé comme suit.

Le milieu réactionnel est refroidi à 10°C (à l'expiration des 2 heures). Une quantité de 293 g (16,28 mol) d'eau est alors additionnée en maintenant la température à 10°C. L'ONTA cristallisé est alors filtré puis essoré sur filtre Büchner.

La masse d'ONTA humide récupérée est de 403 g.

L'analyse HPLC (de l'ONTA récupéré) permet d'établir, après étalonnage externe (avec de l'ONTA commercial), un rendement de 96 % en produit humide (/ à l'ATA de départ).

La nature du produit obtenu (ONTA) a aussi été confirmée par RMN ¹³C.
RMN ¹³C (DMSO, 100 MHz): δ 158,22 ppm (s), 139,24 ppm (s).

## Revendications

1. Procédé d'obtention d'une solution de 1,2,4-triazol-5-one (**3**) dans de l'acide sulfurique concentré, **caractérisé en ce qu'**il comprend :
a) la préparation, dans de l'acide sulfurique concentré, de l'hydrogénosulfate de diazonium (**2**) de formule : 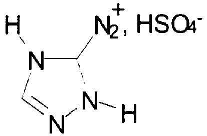 par réaction, à froid, dans de l'acide sulfurique concentré, d'au moins un nitrite sur le 3-amino-1,2,4-triazole (**1**) ; et
b) le traitement thermique du milieu réactionnel renfermant ledit hydrogénosulfate de diazonium (**2**) pour l'obtention de ladite solution.

2. Procédé selon la revendication 1, **caractérisé en ce que** ledit acide sulfurique concentré renferme au moins 75 % en masse, avantageusement au moins 80 % en masse, très avantageusement au moins 95 % en masse, d'acide sulfurique.

3. Procédé selon la revendication 1 ou 2, **caractérisé en ce que** la concentration dudit 3-amino-1,2,4-triazole (**1**) dans l'acide sulfurique concentré est comprise entre 0,3 et 2 mol/l, avantageusement entre 1,3 et 1,6 mol/l.

4. Procédé selon l'une quelconque des revendications 1 à 3, **caractérisé en ce que** ledit au moins un nitrite est choisi parmi les nitrites organiques, les nitrites d'alcalins et les nitrites d'alcalino-terreux, **en ce que** ledit au moins un nitrite consiste avantageusement en le nitrite de sodium (NaNO₂) et/ou le nitrite de potassium (KNO₂), très avantageusement en le nitrite de sodium (NaNO₂).

5. Procédé selon l'une quelconque des revendications 1 à 4, **caractérisé en ce que** le rapport molaire nitrite(s) / 3-amino-1,2,4-triazole (**1**) est compris entre 1 et 2, avantageusement entre 1,2 et 1,4.

6. Procédé selon l'une quelconque des revendications 1 à 5, **caractérisé en ce que** ledit au moins un nitrite est mis à réagir sur ledit 3-amino-1,2,4-triazole (**1**) à une température entre 0°C et 10°C, avantageusement entre 2°C et 7°C.

7. Procédé selon l'une quelconque des revendications 1 à 6, **caractérisé en ce que** ledit traitement thermique comprend deux phases :
- une première phase de montée en température, mise en oeuvre avec contrôle du dégagement de gaz, de façon à éviter tout débordement, jusqu'à une température T d'au moins 50°C, avantageusement en deçà de 120°C, très avantageusement jusqu'à une température T de 100°C, et
- une seconde phase de maintien à ladite température T.

8. Procédé selon la revendication 7, **caractérisée en ce que** la montée en température de la première phase est mise en oeuvre selon une rampe de 9°C/h à 16°C/h, avantageusement selon une rampe de 12°C/h.

9. Solution de 1,2,4-triazol-5-one (**3**) dans de l'acide sulfurique concentré, susceptible d'être obtenue par le procédé selon l'une quelconque des revendications 1 à 8, ladite solution renfermant au moins un hydrogénosulfate, autre que l'hydrogénosulfate de diazonium (**2**).

10. Procédé de préparation du 3-nitro-1,2,4-triazol-5-one (**4**), **caractérisé en ce qu'**il comprend :
a) la mise à disposition d'une solution de 1,2,4-triazol-5-one (**3**) dans de l'acide sulfurique concentré selon la revendications 9 ou l'obtention d'une solution de 1,2,4-triazol-5-one (**3**) dans de l'acide sulfurique concentré selon le procédé de l'une quelconque des revendications 1 à 8 ; puis
β) la nitration dudit 1,2,4-triazol-5-one (**3**) par addition d'acide nitrique concentré à la dite solution.

11. Procédé selon la revendication 10, **caractérisé en ce que** ledit acide nitrique concentré renferme au moins 75 % en masse, avantageusement à au moins 80 % en masse, très avantageusement à au moins 95 % en masse, d'acide nitrique.

12. Procédé selon la revendication 10 ou 11, **caractérisé en ce que** ledit acide nitrique concentré est ajouté en un rapport molaire HNO₃ / 1,2,4-triazol-5-one (**3**) compris entre 1,9 et 2,5, avantageusement entre 1,95 et 2,2.

13. Procédé selon l'une quelconque des revendications 10 à 12, **caractérisé en ce que**, 10 à 20 %, avantageusement 15 %, en masse d'eau (H₂O) sont ajoutés à ladite solution, avant ladite addition d'acide nitrique concentré.

14. Procédé selon l'une quelconque des revendications 10 à 13, **caractérisé en ce qu'**il comprend :
- une première étape d'obtention d'une solution du 1,2,4-triazol-5-one (3) dans de l'acide sulfurique concentré selon le procédé de l'une quelconque des revendications 1 à 8, et
- une seconde étape de nitration dudit 1,2,4-triazol-5-one (**3**) par addition d'acide nitrique concentré à la dite solution ;
lesdites première et seconde étapes étant mises en oeuvre au sein d'un même réacteur.

15. Procédé selon l'une quelconque des revendications 10 à 14, **caractérisé en ce qu'**il comprend en outre la récupération du 3-nitro-1,2,4-triazol-5-one (**4**) ; ladite récupération comprenant une précipitation de celui-ci par refroidissement du milieu réactionnel obtenu à l'issue de la nitration ; de 8 à 15 %, de préférence 10 %, en masse d'eau (H₂O) étant avantageusement ajouté audit milieu réactionnel refroidi pour une précipitation sélective dudit 3-nitro-1,2,4-triazol-5-one (**4**).

16. Procédé selon la revendication 15, **caractérisé en ce que** le refroidissement est mis en oeuvre à une température comprise entre 8 et 12 °C, avantageusement à 10°C.

## Patentansprüche

1. Verfahren zur Herstellung einer Lösung von 1,2,4-Triazol-5-on (3) in konzentrierter Schwefelsäure, **dadurch gekennzeichnet, dass** es aufweist:
a) das Herstellen in konzentrierter Schwefelsäure von Diazoniumhydrogensulfat (2) der Formel: 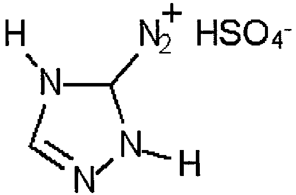 durch Kaltreaktion in konzentrierter Schwefelsäure von mindestens einem Nitrit auf dem 3-Amino-1,2,4-triazol (1) und
b) die Wärmebehandlung des Reaktionsmediums, das das Diazoniumhydrogensulfat (2) enthält, um die Lösung zu erhalten.

2. Verfahren gemäß Anspruch 1, **dadurch gekennzeichnet, dass** die konzentrierte Schwefelsäure mindestens 75 Massenprozent, vorteilhafterweise mindestens 80 Massenprozent, insbesondere mindestens 95 Massenprozent Schwefelsäure enthält.

3. Verfahren gemäß Anspruch 1 oder 2, **dadurch gekennzeichnet, dass** die Konzentration des 3-Amino-1,2,4-triazols (1) in konzentrierter Schwefelsäure zwischen 0,3 und 2 mol/l, vorzugsweise zwischen 1,3 und 1,6 mol/l beträgt.

4. Verfahren gemäß einem der Ansprüche 1 bis 3, **dadurch gekennzeichnet, dass** das mindestens eine Nitrit ausgewählt wird aus den organischen Nitriten, den alkalischen Nitriten und den Erdalkalinitriten und dadurch, dass das mindestens eine Nitrit vorteilhafterweise aus Natriumnitrit (NaNO₂) und/oder Kaliumnitrit (KNO₂), insbesondere aus Natriumnitrit (NaNO₂) besteht.

5. Verfahren gemäß einem der Ansprüche 1 bis 4, **dadurch gekennzeichnet, dass** das Molverhältnis von Nitrit(en)/3-Amino-1,2,4-triazol (1) zwischen 1 und 2, vorteilhafterweise zwischen 1,2 und 1,4 liegt.

6. Verfahren gemäß einem der Ansprüche 1 bis 5, **dadurch gekennzeichnet, dass** das mindestens eine Nitrit auf einem 3-Amino-1,2,4-triazol (1) bei einer Temperatur zwischen 0 °C und 10 °C, vorzugsweise zwischen 2 °C und 7 °C, zur Reaktion gebracht wird.

7. Verfahren gemäß einem der Ansprüche 1 bis 6, **dadurch gekennzeichnet, dass** die Wärmebehandlung zwei Phasen aufweist:
- eine erste Phase des Temperaturanstiegs, die unter Kontrolle der Gasentwicklung derart durchgeführt wird, um jegliches Überlaufen zu verhindern, bis zu einer Temperatur T von mindestens 50 °C, vorteilhafterweise unter 120 °C, insbesondere bis zu einer Temperatur T von 100 °C, und
- eine zweite Phase des Haltens bei der Temperatur T.

8. Verfahren gemäß Anspruch 7, **dadurch gekennzeichnet, dass** der Temperaturanstieg der ersten Phase mit einer Steigung von 9 °C/h bis 16 °C/h, vorteilhafterweise mit einer Steigung von 12 °C/h durchgeführt wird.

9. Lösung von 1,2,4-Triazol-5-on (3) in konzentrierter Schwefelsäure, die nach dem Verfahren gemäß einem der Ansprüche 1 bis 8 erhalten werden kann, wobei die Lösung mindestens ein anderes Hydrogensulfat als Diazoniumhydrogensulfat (2) enthält.

10. Verfahren zur Herstellung von 3-Nitro-1,2,4-triazol-5-on (4), **dadurch gekennzeichnet, dass** es aufweist:
a) das Bereitstellen einer Lösung von 1,2,4-Triazol-5-on (3) in konzentrierter Schwefelsäure gemäß Anspruch 9 oder das Erhalten einer Lösung von 1,2,4-Triazol-5-on (3) in konzentrierter Schwefelsäure nach dem Verfahren gemäß einem der Ansprüche 1 bis 8 und dann
β) das Nitrieren des 1,2,4-Triazol-5-ons (3) durch Zugabe von konzentrierter Salpetersäure zu der Lösung.

11. Verfahren gemäß Anspruch 10, **dadurch gekennzeichnet, dass** die konzentrierte Salpetersäure mindestens 75 Massenprozent, vorteilhafterweise mindestens 80 Massenprozent, insbesondere mindestens 95 Massenprozent Salpetersäure enthält.

12. Verfahren gemäß Anspruch 10 oder 11, **dadurch gekennzeichnet, dass** die konzentrierte Salpetersäure in einem Molverhältnis von HNO₃/1,2,4-Triazol-5-on (3) zugesetzt wird, das zwischen 1,9 und 2,5, vorteilhafterweise zwischen 1,95 und 2,2 liegt.

13. Verfahren gemäß einem der Ansprüche 10 bis 12, **dadurch gekennzeichnet, dass** der Lösung vor der Zugabe von konzentrierter Salpetersäure 10 bis 20 Massenprozent, vorteilhafterweise 15 Massenprozent Wasser (H₂O) zugesetzt werden.

14. Verfahren gemäß einem der Ansprüche 10 bis 13, **dadurch gekennzeichnet, dass** es aufweist:
- einen ersten Schritt des Erhaltens einer Lösung von 1,2,4-Triazol-5-on (3) in konzentrierter Schwefelsäure nach dem Verfahren gemäß einem der Ansprüche 1 bis 8 und
- einen zweiten Schritt des Nitrierens des 1,2,4-Triazol-5-ons (3) durch Zugabe von konzentrierter Salpetersäure zu der Lösung,
wobei der erste und zweite Schritt in demselben Reaktor durchgeführt werden.

15. Verfahren gemäß einem der Ansprüche 10 bis 14, **dadurch gekennzeichnet, dass** es ferner das Rückgewinnen von 3-Nitro-1,2,4-triazol-5-on (4) aufweist, wobei das Rückgewinnen ein Ausfällen von diesem durch Abkühlen des Reaktionsmediums aufweist, das nach Abschluss des Nitrierens erhalten wird, wobei 8 bis 15 Massenprozent, vorzugsweise 10 Massenprozent Wasser (H₂O) vorteilhafterweise zu dem gekühlten Reaktionsmedium für ein selektives Ausfällen des 3-Nitro-1,2,4-triazol-5-ons (4) zugesetzt werden.

16. Verfahren gemäß Anspruch 15, **dadurch gekennzeichnet, dass** das Abkühlen bei einer Temperatur zwischen 8 und 12 °C, vorzugsweise bei 10 °C durchgeführt wird.

## Claims

1. A process for producing a solution of 1,2,4-triazol-5-one (**3**) in concentrated sulfuric acid, **characterized in that** it comprises:
a) the preparation, in concentrated sulfuric acid, of the diazonium hydrogensulfate (**2**) of formula: 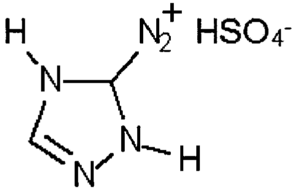 by reaction, under cold conditions, in concentrated sulfuric acid, of at least one nitrite with 3-amino-1,2,4-triazole (**1**); and
b) the heat treatment of the reaction medium including said diazonium hydrogensulfate (**2**) in order to produce said solution.

2. The process as claimed in claim 1, **characterized in that** said concentrated sulfuric acid includes at least 75% by weight, advantageously at least 80% by weight and very advantageously at least 95% by weight of sulfuric acid.

3. The process as claimed in claim 1 or 2, **characterized in that** the concentration of said 3-amino-1,2,4-triazole (**1**) in the concentrated sulfuric acid is between 0.3 and 2 mol/l, advantageously between 1.3 and 1.6 mol/l.

4. The process as claimed in any one of claims 1 to 3, **characterized in that** said at least one nitrite is chosen from organic nitrites, alkali metal nitrites and alkaline earth metal nitrites, **in that** said at least one nitrite advantageously consists of sodium nitrite (NaNO₂) and/or potassium nitrite (KNO₂) and very advantageously of sodium nitrite (NaNO₂).

5. The process as claimed in any one of claims 1 to 4, **characterized in that** the nitrite(s)/3-amino-1,2,4-triazole (**1**) molar ratio is between 1 and 2 and advantageously between 1.2 and 1.4.

6. The process as claimed in any one of claims 1 to 5, **characterized in that** said at least one nitrite is reacted with said 3-amino-1,2,4-triazole (**1**) at a temperature between 0°C and 10°C, advantageously between 2°C and 7°C.

7. The process as claimed in any one of claims 1 to 6, **characterized in that** said heat treatment comprises two phases:
- a first phase of rise in temperature, carried out with control of the release of gas, so as to prevent any overflowing, up to a temperature T of at least 50°C, advantageously below 120°C, very advantageously up to a temperature T of 100°C, and
- a second phase of maintaining at said temperature T.

8. The process as claimed in claim 7, **characterized in that** the rise in temperature of the first phase is carried out according to a gradient of 9°C/h to 16°C/h, advantageously according to a gradient of 12°C/h.

9. A solution of 1,2,4-triazol-5-one (**3**) in concentrated sulfuric acid, obtainable by the process as claimed in any one of claims 1 to 8, said solution containing at least one hydrogensulfate, other than the diazonium hydrogensulfate.

10. A process for the preparation of 3-nitro-1,2,4-triazol-5-one (**4**), **characterized in that** it comprises:
a) making available a solution of 1,2,4-triazol-5-one (**3**) in concentrated sulfuric acid as claimed in claim 9 or producing a solution of 1,2,4-triazol-5-one (**3**) in concentrated sulfuric acid according to the process as claimed in any one of claims 1 to 8; then
β) nitrating said 1,2,4-triazol-5-one (**3**) by addition of concentrated nitric acid to said solution.

11. The process as claimed in claim 10, **characterized in that** said concentrated nitric acid includes at least 75% by weight, advantageously at least 80% by weight and very advantageously at least 95% by weight of nitric acid.

12. The process as claimed in claim 10 or 11, **characterized in that** said concentrated nitric acid is added in an HNO₃/1,2,4-triazol-5-one (**3**) molar ratio of between 1.9 and 2.5, advantageously between 1.95 and 2.2.

13. The process as claimed in any one of claims 10 to 12, **characterized in that** from 10 to 20%, advantageously 15%, by weight of water (H₂O) is added to said solution, before said addition of concentrated nitric acid.

14. The process as claimed in any one of claims 10 to 13, **characterized in that** it comprises:
- a first stage of producing a solution of 1,2,4-triazol-5-one (**3**) in concentrated sulfuric acid according to the process of any one of claims 1 to 8, and
- a second stage of nitrating said 1,2,4-triazol-5-one (**3**) by addition of concentrated nitric acid to said solution;
said first and second stages being carried out within one and the same reactor.

15. The process as claimed in any one of claims 10 to 14, **characterized in that** it additionally comprises the recovery of the 3-nitro-1,2,4-triazol-5-one (**4**), said recovery comprising a precipitation of the latter by cooling the reaction medium obtained on conclusion of the nitration; from 8 to 15%, preferably 10%, by weight of water (H₂O) being advantageously added to said cooled reaction medium for selective precipitation of said 3-nitro-1,2,4-triazol-5-one (**4**).

16. The process as claimed in claim 15, **characterized in that** the cooling is carried out to a temperature of between 8 and 12°C, advantageously to 10°C.
